# EUROPEAN PATENT SPECIFICATION

(11) **EP 3 500 163 B1**
(45) Date of publication and mention of the grant of the patent: **19.02.2025**
(21) Application number: 17752130.9
(22) Date of filing: 15.08.2017
(51) Int. Cl.: G16H 20/30, G16H 40/67, A61B 5/021, A61B 5/00

(54) **BLOOD-PRESSURE MANAGEMENT**
BLUTDRUCKVERWALTUNG
GESTION DE LA PRESSION ARTÉRIELLE

(30) Priority: 18.08.2016 EP 16184787
(43) Date of publication of application: 26.06.2019
(73) Proprietor: Koninklijke Philips N.V., 5656 AG Eindhoven (NL)
(72) Inventor: RADHA, Mustafa, Ghassan, 5656 AE Eindhoven (NL); MENA BENITO, Maria, Estrella, 5656 AE Eindhoven (NL); VAN EE, Raymond, 5656 AE Eindhoven (NL); DESSAUD, Nathalie, Magali, Danielle, 5656 AE Eindhoven (NL); HELAOUI, Rim, 5656 AE Eindhoven (NL); SPEKOWIUS, Gerhard, Reinhold, 5656 AE Eindhoven (NL)
(74) Representative: Philips Intellectual Property & Standards
(86) International application number: PCT/EP2017/070688
(87) International publication number: WO 2018/033546

(56) References cited:
- WO-A1-2015/162513
- US-A1- 2009 105 560
- US-A1- 2010 267 520
- US-A1- 2013 296 723
- H. JONES ET AL: "Exercise Intensity and Blood Pressure During Sleep", INTERNATIONAL JOURNAL OF SPORTS MEDICINE, vol. 30, no. 02, 10 September 2008 (2008-09-10), DE, pages 94 - 99, XP055350196, ISSN: 0172-4622, DOI: 10.1055/s-2008-1038742
- S PARK ET AL: "Time of day for exercise on blood pressure reduction in dipping and nondipping hypertension", JOURNAL OF HUMAN HYPERTENSION, vol. 19, no. 8, 16 June 2005 (2005-06-16), GB, pages 597 - 605, XP055348678, ISSN: 0950-9240, DOI: 10.1038/sj.jhh.1001901

## Description

### FIELD OF THE INVENTION

This invention relates to blood-pressure management, and more particular to management of a blood-pressure variation of a patient.

### BACKGROUND OF THE INVENTION

It is widely known that the blood pressure (BP) of a person typically drops or "dips" to a minimum value while the person is sleeping, a process driven by the diurnal biological rhythm. This reduction or dipping in BP is commonly defined as the ratio of the average sleeping (e.g. night-time or nocturnal) blood pressure to average waking (e.g. day-time) blood pressure, and it can occur in both systolic blood pressure (SBP), diastolic blood pressure (DBP) and mean arterial (MAP) blood pressure values.

Such BP dipping is commonly categorized into the following four segments or categories:
(i) Absence of dipping: ratio larger than 1;
(ii) Mild dipping: ratio between 0.9 and 1;
(iii) Dipping: ratio between 0.8 and 0.9; and
(iv) Extreme dipping: ratio lower than 0.8

The absence of a decrease in sleeping blood pressure (BP) relative to waking BP is commonly referred to a "non-dipping" and it is associated with various health problems such as sleep-related problems (sleep disordered breathing (SDB) or insomnia, for example), obstructive sleep apnea (OSA), obesity, orthostatic hypotension, autonomic dysfunction, chronic kidney disease (CKD), diabetic neuropathy and old age. It has also been found to be an overall predictor of fatal and non-fatal mortality. Also, extreme dipping is associated with various health problems. BP dipping is therefore an important risk factor that should preferably be monitored and/or managed.

Known blood-pressure management or control concepts typically rely on the timed application (e.g. ingestion) of anti-hypertensive medication. However, such medication may have the drawbacks of being expensive, impractical and/or causing undesirable side-effects. Thus, there exists a need for BP monitoring and/or management concepts that are preferably non-invasive and/or don't require the administration of drugs/medication. Such non-invasive monitoring and/or drug-free concepts may be beneficial for enhanced diagnostics, therapy planning or general health in relation to many medical conditions, including CKD, sleep-related problems, OSA, obesity, and the like.

"Exercise Intensity and Blood Pressure During Sleep" by H Jones et al. and "Time of day for exercise on blood pressure reduction in dipping and nondipping hypertension" by S Park et al disclose effects of exercise on blood pressure.

US 2010/0267520 A1 discloses a method of estimating stress caused by an exercise that includes receiving questionnaire data including body measurements from a user, measuring health measurement data, deducing an exercise target and an exercise prescription based on the received and measured data, and transmitting the exercise target and exercise prescription to the user. The questionnaire data is then received back from the user, including the body measurements from the user and measuring again the health measurements data; parameters are designed based on the received and re-received questionnaire data and the measured and remeasured health measurement data and the parameters are converted to predetermined values. A regression analysis model is designed for estimating the exercise stress using the parameters and performing regression analysis and the exercise stress estimated through the regression analysis is transmitted to the user.

### SUMMARY OF THE INVENTION

The invention aims to at least partly fulfil one of the aforementioned needs. To this end, the invention provides devices, methods and computer program products as defined in the independent claims. The dependent claims provide advantageous embodiments. Thus, the invention provides an apparatus and a corresponding method for managing a blood-pressure variation (e.g. BP dipping) of a patient as defined in the independent claims.

Thus, there are proposed alternative, medication-free exercise-based concepts for managing or controlling BP dipping (including non-dipping or extreme dipping) through the combined timing and intensity of physical exercise. Such BP dipping typically occurs during sleep and so it is often referred to a nocturnal BP dipping or sleeping BP dipping.

Proposed embodiments are based on using the acute temporary anti-hypertensive benefits of exercise (known as post-exercise hypotension). Such benefits may also be combined with the finding that exercise-related signals in the brain also facilitate the synchronization of the internal circadian clock with the day-night cycle, improving the circadian rhythm. It is proposed to provide a personalised approach which prescribes exercise to a patient, wherein the timing and intensity of the exercise is set based on a measured BP dip of the patient and the patient's circadian rhythm (e.g. internal clock). In particular, embodiments may determine a prescribed (e.g. preferred or target) exercise intensity for an individual based on an obtained measure of a diurnal (e.g. sleeping) BP dip of the individual. Further, embodiments may determine a prescribed (e.g. preferred, advised or target) exercise timing for the individual based on an obtained measure of the individual's circadian rhythm. Based on the prescribed timing and intensity of exercise, a deficiency in the individual's BP dip may be resolved for example.

Proposed embodiments may therefore leverage information about an individual's BP variation(s) over the course of a predetermined time period (such as a day for example) in combination with information about the individual's circadian (e.g. 24-hour) rhythm.

In this way, there may be provided a tool for managing a value of BP dipping that can be used by a medical professional, a general practitioner, or (medically) un-trained individual, for example. This may alleviate a need for close monitoring by medical professionals. It may also reduce a need for medical intervention or treatment. Embodiments may therefore relieve healthcare requirements/resources. Embodiments may also assist in the supervision or management of a patient at risk of health complications due to abnormal BP variations. Similarly, it may help as a preventative measure for persons who are at risk of developing health problems. Embodiments may also be used to verify the success of a prescribed activity or exercise and/or to monitor BP dipping progression or exacerbation.

The step of determining a target exercise timing for the patient comprises, as one option: determining a predicted timing of melatonin onset for the patient based on the measure of the circadian rhythm; and processing the predicted timing of melatonin onset in accordance with one or more algorithms to calculate the target exercise timing for the patient. In this way, embodiments may account for the patient's sleep pattern or expected time of sleeping so as to determine an optimal time for undertaking exercise to address a BP dip issue.

The step of determining a target exercise timing for the patient comprises, as another option, comparing the measure of circadian rhythm with a threshold value. Based on the result of the comparison, the target exercise timing may be determined to either be in a time window after waking or a time window before sleeping. For instance, embodiments may be adapted to determine whether a person is a 'morning person' or 'evening person' by comparing their internal diurnal rhythm to the world's day/night rhythm. For an evening person, the circadian rhythm may be compared with a first threshold value corresponding to melatonin onset time so as to derive a recommended exercise time window before sleep. For a morning person, the circadian rhythm may be compared with a second threshold value corresponding to a morning awakening so as to derive a recommended exercise time window after waking. Embodiments may thus be tailored to the specific circadian rhythm of a patient in order to provide optimal exercise prescriptions for managing BP variations of the patient.

The step of determining a target exercise intensity for the patient comprises: obtaining a waking blood pressure of the patient; calculating a target sleeping blood pressure for the patient based on the waking blood pressure and the measure of diurnal blood pressure dip of the patient; and calculating the target exercise intensity using an algorithm based on the target sleeping blood pressure. In this way, a BP differential between waking and sleeping may be obtained and then used to determine an optimal or preferred (i.e. target) sleeping BP for a patient. Using known or established relationships between exercise intensity and resultant BP variation, an exercise intensity for causing a BP variation to meet the optimal or preferred (i.e. target) sleeping BP may be determined.

Also, an embodiment may further comprise: obtaining an updated measure of a diurnal blood pressure dip of the patient, the updated measure being associated with a previously determined target exercise intensity; and modifying the algorithm based on the updated measure of a diurnal blood pressure dip of the patient and the previously determined target exercise intensity. In this way, the algorithm or function with which the target exercise intensity is calculated may be adapted (e.g. updated or calibrated) based on an actual observed result obtained using the target exercise intensity. For example, if one considers an example wherein, on day 1, the target exercise intensity is determined to be 50% to achieve a blood pressure decrease of 5 mmHg, a new or updated measurement may be obtained which indicates that, on day 2, the achieved blood pressure decrease was actually 7 mmHg. The algorithm may then be adapted to propose a slightly lower exercise intensity in order to achieve a 5 mmHg decrease, since the updated measure indicates that the user has a stronger exercise response than the average person for example Such embodiments may therefore dynamically and automatically adapt to implementation specifics and/or characteristics of individuals.

Embodiments may further comprise sensing, with a sensor arrangement worn by or coupled to the patient, a value of at least one of: body temperature of the patient; activity of the patient; respiration rate of the patient; respiration rate variability of the patient; pulse rate of the patient; and pulse rate variability of the patient. A sensor output signal may then be generated based on the sensed value(s). Various physiological values or parameters of the patient may thus be obtained for the purpose of determining the circadian rhythm of the patient. One or more different approaches to determining the circadian rhythm may therefore be implemented, and such approaches may be combined for improved accuracy. Also, the ability to use various approaches to determine the circadian rhythm of the patient may enable embodiments to be adapted to various constraints, such as available resources, cost or complexity for example.

Further, embodiments may comprise receiving, at a processing unit, a sensor output signal from the sensor arrangement. The received sensor output signal may then be processed in accordance with one or more data processing algorithms to determine the measure of the circadian rhythm of the patient. Embodiments may further comprise sensing, with a BP sensing arrangement: a waking BP of the patient when awake; and a sleeping BP of the patient when asleep. A blood pressure output signal may then be generated based on the sensed value(s). Embodiments may therefore employ one or more sensors to obtain BP information about a patient and use this information for determining the diurnal BP dip of the patient. Alternative embodiments may, however, receive information regarding a measure of a diurnal BP dip of the patient from a separate or external method. Embodiments may therefore be modular in nature, or complete/contained methods that are adapted to sense/detect the information that may be needed for determining a BP dip and/or a circadian rhythm.

Proposed embodiments may further comprise receiving, at a processing unit, a blood pressure output signal from the blood pressure sensing arrangement. The received blood pressure output signal may then be processed in accordance with one or more data processing algorithms to determine the measure of the diurnal blood pressure dip of the patient.

Embodiments may therefore take account of a context of the patient, such as their current activity or physical properties for example.

Proposed embodiments may further comprise generating instructions for displaying a GUI on a display device using a processor device, wherein the graphical user interface is adapted to communicate information about the target exercise intensity and target exercise timing to a user. Generating instructions for display of a GUI can mean generating a control signal for use by a display device. Such instructions can be in the form of simple images such as bitmap JPEG or other format. However, such instructions can also be more complex allowing real time build-up of the GUI or parts of the GUI on a regular display device such as for example CRT, LCD, OLED, E-ink or the like.

According to another aspect, there may be provided a computer program product downloadable from a communications network and/or stored on a computer readable medium and/or microprocessor-executable medium wherein the computer program product comprises computer program code instructions, which when executed by at least one processor, implement a method according to a proposed embodiment.
For example, by the apparatus according to the invention a user may be advised of a recommended timing and intensity to exercise at.

The exercise timing calculation unit may be adapted to: determine a predicted timing of melatonin onset for the patient based on the measure of the circadian rhythm; and process the predicted timing of melatonin onset in accordance with one or more algorithms to calculate the target exercise timing for the patient or may be adapted to: compare the measure of circadian rhythm with a threshold value; and based on the result of the comparison, determine the target exercise timing to either be in a time window after waking or a time window before sleeping.

The exercise intensity calculation unit is adapted to: obtain a waking blood pressure of the patient; calculate a target sleeping blood pressure for the patient based on the waking blood pressure and the measure of diurnal blood pressure dip of the patient; and calculate the target exercise intensity based on the target sleeping blood pressure.

Embodiments may further comprise a sensor arrangement adapted to be worn by or coupled to the patient, and to sense a value of at least one of: body temperature of the patient; activity of the patient; respiration rate of the patient; respiration rate variability of the patient; pulse rate of the patient; and pulse rate variability of the patient. The sensor arrangement may also generate a sensor output signal based on the sensed value(s).

In an embodiment, the apparatus may further comprise: a blood pressure sensing arrangement adapted to sense: a waking blood pressure of the patient when awake; and a sleeping blood pressure of the patient when asleep; and to generate a blood pressure output signal based on the sensed value(s).

Embodiments may further comprise a user input interface adapted to receive a user input signal representative of at least one of: patient information; and a limit value representative of an acceptable limit of a BP variation (e.g. BP dipping). Embodiments may therefore be thought of as providing an interface which enables a user to further specify information or data that may be relevant for the purpose of determining or monitoring a BP variation(s) and/or prescribed exercise timing and intensity. Such user-specified information may enable unique traits, circumstances and/or conditions specific to the user or the environment to be accounted for when determining or monitoring BP variation(s) and/or prescribed exercise timing and intensity.

Thus, there may be provided a tool which enables a user to further specify factors to be included in the determination or prescribed exercise timing and intensity., e.g. by specifying a value or value range for a user attribute or activity. Embodiments may therefore provide input options, increasing the flexibility and power of BP dipping management.

In some embodiments, the apparatus may further comprise a communication interface adapted to communicate with one or more databases so as to obtain at least one of the information that may be used in determining or monitoring BP variation(s) and/or prescribed exercise timing and intensity.

There may be provided a portable computing device comprising apparatus for managing BP variation of a patient according to a proposed embodiment.

The system may further comprise a display device for displaying a graphical or non-graphical (e.g. auditory) user interface, wherein the graphical user interface is adapted to communicate information about prescribed exercise timing and intensity of a patient to a user.

Embodiments may comprise a client device comprising a data processor device. This may be a standalone device adapted to receive information from one or more remotely positioned information sources (via a communication link for example) and/or even adapted to access information stored in a database for example. In other words, a user (such as a medical professional, technician, researcher, patient etc.) may have an appropriately arranged client device (such as a laptop, tablet computer, mobile phone, PDA, etc.) which provides a system according to an embodiment and thus enables the user to provide data or information for the purpose of managing BP variation of a patient.

The system may comprise: a server device comprising the at least one processor, where the server device may be configured to transmit generated instructions for determining and/or displaying a BP variation and/or prescribed exercise timing and intensity to a client device or communication network. In such a configuration, display instructions are made available by a server. A user may therefore link with the server to work with the system.

The processor may be remotely located from the display device, and a control signal may thus be communicated to the display device via a communication link. Such a communication link can be e.g. the internet and/or a wireless communication link. Other suitable short-range or long-range communication links and/protocols may be employed. In this way, a user (such as a medical researcher, general practitioner, data analyst, engineer, patient etc.) may have an appropriately arranged device that can receive and process information according to an embodiment for managing a BP variation of a patient. Embodiments may therefore enable a user to remotely managing a BP variation of a patient using a portable computing device, such as a laptop, tablet computer, mobile phone, PDA, etc. Embodiments may also enable data retrieval after a monitored time period.

The system may further comprise: a server device comprising the at least one processor; and a client device comprising a display device. Dedicated data processing means may therefore be employed for the purpose managing a BP variation of a patient, thus reducing processing requirements or capabilities of other components or devices of the system.

Thus, it will be understood that processing capabilities may be distributed throughout the system in different ways according to predetermined constraints and/or availability of processing resources.

According to another aspect of the invention, there may be provided a portable computing device comprising apparatus for managing a blood-pressure variation of a patient according to proposed embodiments. For example, embodiments may be implemented in a wearable computing device, such as a smart watch or fitness tracker for example. In this way, the apparatus may be suitable positioned with respect to a patient so as to enable the gathering/obtaining of patient information that may be used for purpose of determining a diurnal blood pressure dip and/or a circadian rhythm of the patient. The portable computing device may, for example, be designed such the one or more sensors of the device can contact a carrier or wearer of the device.

Embodiments may provide concepts for managing BP variations of an individual/person. The proposed concepts may comprise determining both an intensity and timing of exercise to be undertaken by the individual so as to manage a diurnal BP variation of the individual (e.g. a BP dip when the individual is sleeping). Recommended exercise intensity may be calculated and prescribed for the individual based on measure of a diurnal BP dip which occurred when the individual slept. A recommended timing of the exercise may be calculated and prescribed for the individual based on measure of the circadian rhythm of the individual. Such prescribed exercise intensity and timing may then be communicated to the individual so as to instruct how to influence BP in a positive manner. For this purpose, proposed concepts may employ (or be employed on) at least one processor.

It will be understood that proposed embodiments may be applicable for any personal health programs or applications targeting blood pressure dipping. In particular, embodiments may be of particular benefit to individuals or patients who experience disrupted blood pressure dipping (e.g. non-dipping) such as shift worker and/or people suffering from sleep apnoea.

The independent claims define analogous advantages features for method and system/apparatus claims. The advantages explained for the method herein above and herein below may therefore also apply to the corresponding apparatus.

### BRIEF DESCRIPTION OF THE DRAWINGS

The invention will now be described in detail with reference to the following schematic drawings:
Fig. 1 illustrates apparatus for managing a blood-pressure variation of a patient according to an embodiment;
Fig. 2 is a flow diagram of a managing a blood-pressure variation of a patient according to an embodiment; and
Fig. 3 is a flow diagram of a managing a blood-pressure variation of a patient according to an embodiment; and
Fig. 4 illustrates an example of a computer within which one or more parts of an embodiment may be employed.

### DETAILED DESCRIPTION OF THE EMBODIMENTS

Proposed embodiments relate to approaches and tools for managing BP variations or dipping of a patient.

In particular, it is proposed to leverage the understanding that physical exercise leads to a predictable, acute and temporary decrease in blood pressure of an individual. For example, physical exercise can acutely decrease the BP of an individual for a period of at least nine hours after the physical exercise. This decrease is known as post-exercise hypotension (PEH).

It has been identified that the effect of PEH on sleeping (e.g. nocturnal) blood pressure is dependent on a number of parameters including baseline BP, exercise intensity, exercise timing, and blood markers.

### Baseline Blood Pressure

It has been found that the magnitude of PEH becomes stronger as baseline blood pressure increases. In subjects with a healthy blood pressure, PEH will be rather limited. In subjects with a high blood pressure, the effect of PEH can be strong enough to lower the blood pressure to healthy levels for a specific duration.

### Exercise Intensity

A linear dose-response relationship between the exercise intensity and the magnitude of PEH has been identified. For example, it has been found that an individual may experience a decrease of 1.5 mmHg in systolic BP per 10% increase in oxygen uptake during exercise (relative to the maximum oxygen uptake).

### Exercise Timing

In order for the BP lowering effects of PEH to affect the sleeping (e.g. nocturnal) BP, it is proposed that the exercise should be timed within a certain time window. By way of example, it has been identified that exercise in the evening will have a larger impact on the BP dip than exercise during the afternoon. However, this may need to be balanced against the consideration that exercise in the evening may not be undertaken very late in the night as it may have an adverse effect on sleep onset latency (and may also cause further complications in subpopulations, such as hot flashes in menopausal women for example). Accordingly, there may be a specific time window in which it is beneficial to exercise in the evening.

### Blood Markers

Some blood markers (glucose, cholesterol, etc.) have been found to correlate with the magnitude of PEH.

Taking account of the abovementioned parameters, it is proposed to manage or control BP dipping of an individual by determining both timing and intensity of physical exercise for the individual. In particular, it is proposed to provide a tailored approach which prescribes exercise to a patient, wherein the timing and intensity of the exercise is prescribed based on a measured BP dip of the patient and the patient's circadian rhythm (e.g. internal clock). More specifically, optimal or target exercise intensity is determined based on an obtained measure of a diurnal BP pressure dip of the individual. Further, an optimal or target timing of the exercise is determined based on an obtained measure of the individual's circadian rhythm. The determined values for exercise timing and intensity may then be adhered to by the individual so as to resolve an abnormal diurnal BP pressure dip for example.

Embodiments are therefore based on using the diurnal BP dip and circadian rhythm of an individual to recommend an intensity and time of exercise to be performed. This approach leverages the BP lowering effects of PEH combined with their time dependent nature so as to cater for a patient's circadian rhythm.

Embodiments may therefore be utilized to manage BP dipping in individuals and to better control healthcare or support therapy for patients with various health problems.

Also, the proposed invention may provide concepts for managing exercise parameters that can be employed by a general physician or (medically) un-trained person without the support of a trained medical professional. This may alleviate a need for medical professionals and/or medical intervention, thus potentially relieving healthcare requirements/resources.

Some embodiments may employ a sensor arrangement adapted to worn by or coupled to a patient. By way of example, the sensor arrangement may detect a value of at least one of: body temperature of the patient; activity of the patient; respiration rate of the patient; respiration rate variability of the patient; pulse rate of the patient; and pulse rate variability of the patient. Embodiments of the invention may therefore be utilized in conjunction with many different types of sensors and/or information databases that may provide information useful for determining a patient's BP and/or circadian rhythm and which more accurately accounts for the specific attributes of the patient, activity of the patient, and/or surrounding environment. A database may comprise, for instance, data relating to the individual's medical history or data relating to cardio-respiratory parameter values in different environmental conditions. For example, information or data employed by embodiments may comprise patient activity, vital signs, temperature, etc.

Embodiments may therefore provide a method, device and/or system that provides for user-specific management of BP dipping which takes account of contextual factors (including a physical attributes and activity of a patient, for example) in order to provide more accurate management of BP dipping. This may enable measurement and management of BP variations for a specific user, whilst enabling the user to partake in desired activities of daily life. Illustrative embodiments may therefore provide concepts which take account of rules and/or relationships relating to activity and physical attributes of the patient. Dynamic context-based BP dipping management may therefore be provided by proposed embodiments.

As a result, proposed embodiments may be of benefit in health programs or applications targeting blood pressure dipping, especially where users require tailored and/or accurate BP management. One such example may enable patients that are highly susceptible to BP dipping problems to gain a level of independence whilst still managing their potential exposure to BP dipping issues. This may, in turn, improve patient health, hospital efficiency, and available healthcare resources. Embodiments may therefore be of particular benefit for medical applications.

The following description provides a context for the description of elements and functionality of the invention and of how elements of the invention can be implemented.

In the description, the following terms and definitions are used.

BP dipping is the reduction or dipping in BP which is driven by the diurnal biological rhythm and typically occurs when a person sleeps. It can occur in both systolic blood pressure (SBP), diastolic blood pressure (DBP) and mean arterial (MAP) blood pressure values. It is therefore often referred to as circadian, night-time, nocturnal or diurnal BP dipping and measured as the ratio of the average sleeping (e.g. night-time or nocturnal) BP to average waking (e.g. day-time) BP. A "non-dipper" is a person exhibiting an absence of a diurnal BP dip.

A graphical user interface (GUI) is a type of interface that allows users to interact with electronic devices through graphical icons and visual indicators such as secondary notation.

A display device is an electronic display device that can be controlled by a display control device. The display control device can be part of, or operate together with, a processor device.

Generating instructions for displaying a GUI can comprise (or be as simple as) constructing images (Bitmap, JPEG, Tiff or the like) of GUI views to be displayed on a display device using regular methods known in the art. Alternatively, such generation of instructions can comprise more dedicated instructions for real time build-up of a GUI view. The instructions can be in the form of a display control signal.

According to various embodiments, there are proposed several approaches to managing BP variations (such as BP dipping) of a patient.

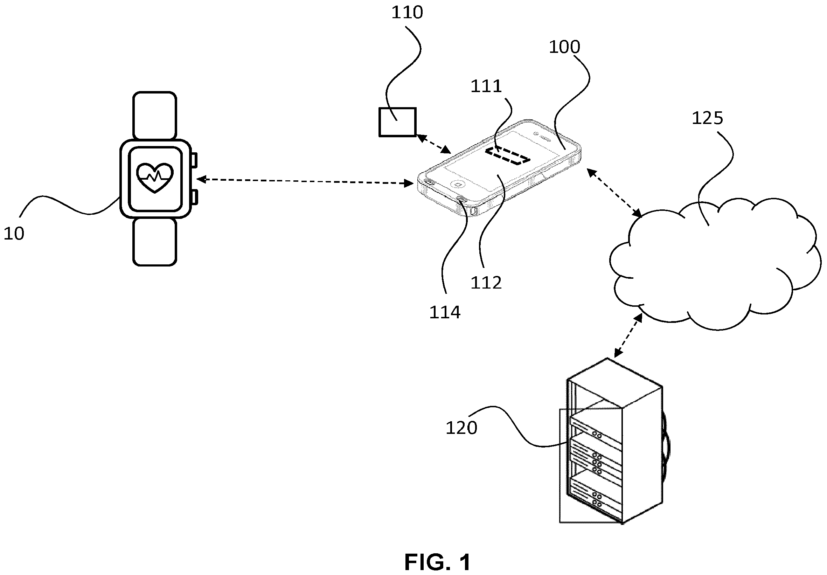

Turning now to Figure 1, there is illustrated an apparatus for managing a blood-pressure variation of a patient according to an embodiment. In such an embodiment, the apparatus comprises a wearable BP sensing device 10 and a processing unit which is integrated in a portable computing device (e.g. a smartphone) 100. Using a built-in communication interface, the portable computing device 100 can receive signals from the wearable BP sensing device 10 and other, supplementary sensors 110 and the process the received signals in accordance with one or more data processing algorithms to determine an exercise timing and intensity for a patient wearing the BP sensing device 10. Further, using the conventional communication abilities of the portable computing device 100, the device can communicate with one or more databases so as to obtain information that may be used in managing a BP variation of the patient. Such information may enable unique traits, circumstances and/or conditions specific to the user or the environment to be accounted for when how to manage a BP variation of the patient.

Also, a display 112 of the portable computing device 100 may be used to display a graphical user interface which communicates information about a determined (e.g. prescribed) exercise timing and intensity to a user of the device 100.

In more detail, the embodiment of Figure 1 comprises a wearable BP sensing device 10 that is integrated in a smartwatch 10 with communication abilities.

Here, the BP sensing device 10 is adapted to be worn by (or coupled to) the patient and a blood pressure sensing arrangement (not visible in Fig. 1) adapted to sense: a (waking) blood pressure of the patient when awake; and a (sleeping) blood pressure of the patient when asleep. The BP sensing device 10 generates a blood pressure output signal based on the sensed value(s). In this way, the BP sensing device 10 is adapted to obtain information about a wearer's (e.g. the patient's) BP, wherein such information comprises (or can be used to determine) a measure of BP variations (e.g. a nocturnal BP pressure dip) of the patient.

The BP sensing device 10 of this embodiment also comprises a supplementary sensor arrangement (not visible in Fig. 1) adapted to sense a value of at least one of: body temperature of the patient; activity of the patient; respiration rate of the patient; respiration rate variability of the patient; pulse rate of the patient; and pulse rate variability of the patient. The BP sensing device 10 is this adapted to sense values that comprise (or can be used to determine) a measure of the circadian rhythm of the patient.

Thus, in the example of Fig. 1, the smartwatch 10 is adapted to obtain data or information (e.g. measurements, values, readings of patient parameters) relating to a BP dip of a patient and a circadian rhythm of the patient.

Based on the obtained data/information, the smartwatch 10 generates a sensor output signal for outputting to a signal/data processing unit, namely the smartphone 100 in the example of Fig. 1.

Thus, in this embodiment, the signal/data processing unit is not integrated into the smartwatch 10, but is instead provided as part of a portable computing device 100 that may be situated near the smartwatch 10 (e.g. within a few metres). Of course, in other embodiments, the signal processing unit may be integrated in the smartwatch 10.

For communicating the sensor output signal to the portable computing device 100, the smartwatch 10 comprises a communication interface (not shown) which is adapted to establish a wireless communication link with the portable computing device 100. Any suitable short-range or long-range communication links and/protocols may be employed.

The portable computing device 100 namely a smartphone 100 (comprising data acquisition and processing components) is adapted to receive information from smartwatch 10 worn or carried by the patient via a wireless communication link.

The received sensor output signals from the smartwatch 10 thus comprises data that may be used (e.g. processed in accordance with an algorithm) so as to obtain a measure of sleeping BP dip of the patient and a measure of the circadian rhythm of the patient. These measures can then be used (e.g. processed in accordance with one or more algorithms) so as to determine a target exercise intensity and target exercise timing.

For example, the smartphone 100 of this example is adapted to implement a data processing algorithm which identifies a predicted timing of melatonin onset for the patient based on a measure of the patient's circadian rhythm. The algorithm then further processes the predicted timing of melatonin onset in accordance to calculate a target exercise timing for the patient. For instance, the processing unit 111 of the smartphone 100 can compare the measure of circadian rhythm with a predetermined threshold value and, based on the result of the comparison, can then determine the target exercise timing to either be in a time window after waking or a time window before sleeping.

Further, the smartphone 100 of this example is also adapted to implement a data processing algorithm which calculates a target sleeping BP for the patient based on an obtained measure of the patient's waking BP and an obtained measure of a diurnal BP dip of the patient. The algorithm then further processes the target sleeping blood pressure to calculate the target exercise intensity.

Furthermore, the smartphone 100 is also adapted to receive information from a supplementary sensor unit 110 which is adapted to sense a value of at least one of: movement; temperature; location, etc. The smartphone 100 is adapted to analyse the obtained measure of BP dip and/or circadian rhythm in combination with the received supplementary sensor 110 output signal(s) to determine at least one of: a refined BP dip and/or circadian rhythm value; an indication of accuracy or reliability; a sleep state of the patient; an activity of the patient; and an indication of event occurrence. In this way, a context of the patient (such as their current activity or physical properties for example) can be taken into account in the process of determining exercise timing and intensity for the patient.

The smartphone 100 is also adapted to send and/or receive information to/from a remotely located server 120 via the Internet 125.

The information obtained by the smartphone 100 is processed to assess and identify factors which may influence the obtained measures of BP dip and/or circadian rhythm of the patient. By way of example, environmental information; patient information; and a limit value representative of an acceptable upper/lower limit of a BP dip may be used in the determination of exercise timing and intensity for a patient.

The information/data processing may be done by the smartphone 100, by the 'Cloud', or by any combination thereof. The embodiment of Figure 1 is therefore implemented as a distributed processing environment in which various types of information/data are processed so as to determine or monitor a cardio-respiratory function of the patient.

The smartphone 100 also comprises an output interface, namely a display 112 and speaker 114 arrangement, adapted to generate an output signal representative of the determined exercise timing and exercise intensity. For example, the user may be advised of timing to undertake a specific exercise program and guided via voice or visual prompts to undertake the exercise program in a manner which attempts to ensure that the correct exercise intensity is employed.

The smartphone 100 is also adapted to receive (e.g. via its touch sensitive screen 112) a user input signal representative of at least one of: environmental information; patient information; and a limit value representative of an acceptable upper/lower limit of a cardio-respiratory value (such as BP, BP dipping heart rate, etc).

The smartphone 100 therefore provides an interface which enables a user to further specify information or data that may be relevant for the purpose of managing BP variations (such as diurnal BP dipping). Such user-specified information enables unique traits, circumstances and/or conditions specific to the user or the environment to be accounted for when managing BP variations. Put another way, the smartphone 100 enables a user to further specify factors to be included in the determination of exercise timing and intensity, e.g. by specifying a value or value range for a user attribute or activity. This provides many input options, increasing the flexibility and power of BP variation management.

Additionally, or alternatively, further environmental information and/or patient information may be provided by other sources or services. For example, local weather conditions and/or medical history data from a database of the server 120 can be used.

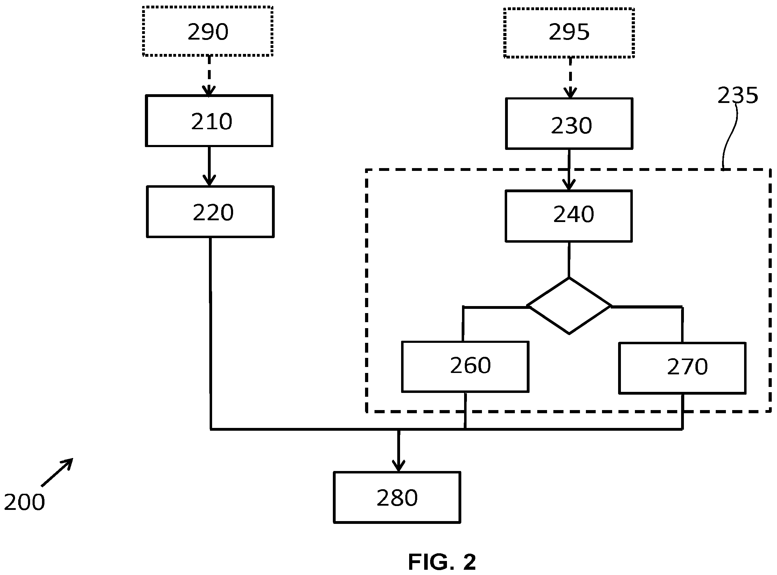

For example, in an exemplary implementation of the system of Figure 2, the server 120 comprises a data processor unit and is configured to transmit generated instructions for determining and/or displaying a determined exercise timing and intensity to a client device or communication network. In such a configuration, display instructions are made available by the server 120. A user of the smartphone 100 can therefore link with the server 120 to work with the system. In this way, data processing means are remotely located from the portable computing device 100, and a control signal can thus be communicated to the portable computing device 100 via a communication link (e.g. the Internet 125).

Accordingly, a user is provided with an appropriately arranged device that can receive and process information relating to a cardio-respiratory function (such as BP) of a patient. Embodiments may therefore enable a user to monitor BP and BP dipping over time using a portable computing device, such as a laptop, tablet computer, mobile phone, PDA, etc. The portable computing device 100 therefore provides a tool that enables the user, for instance, to monitor their cardio-respiratory functions and undertake (or plan) appropriate exercise (to manage BP dipping for example) as they go about their normal daily activities. The user can obtain an understanding of their BP function, which then enables the user to continue or adapt their planned activities (depending on their tolerance to cardio-respiratory issues for example). Also, a medical professional, technician, researcher, etc. may have an appropriately arranged client device (such as a laptop, tablet computer, mobile phone, PDA, etc.) which is adapted to receive information relating to BP function of a monitored user (e.g. patient). In this way, a user can be provided with exercise guidance at a personal level which takes account of the unique attributes and/or activities of the user, and/or the surrounding environment. This alleviates a need for close monitoring by medical professionals or caregivers, for example. It may also reduce a need for medical intervention or treatment.

Dedicated data processing means can therefore be implemented at the server 120 for the purpose of determining a prescribed exercise timing and exercise intensity for a patient, thus relieving or reducing processing requirements at the portable computing device 100.

Thus, it will be understood that processing capabilities may therefore be distributed throughout the system in different ways according to predetermined constraints and/or availability of processing resources of specific embodiments.

Turning now to Figure 2, there is depicted a flow diagram of a method 200 for managing a blood-pressure variation (e.g. BP dipping) of a patient according to an embodiment.

Step 210 comprises obtaining a measure of a diurnal blood pressure dip of the patient. Here, a blood pressure output signal is received (for example, at a processing unit) from a blood pressure sensing arrangement worn by the patient. By way of example, the blood pressure output signal of this embodiment comprises information relating to sensed values of: a waking BP of the patient when awake; and a sleeping BP of the patient when asleep. Such information may be used to determine the diurnal BP dip of the patient, for example by being processed in accordance with one or more data processing algorithms to determine the measure of the diurnal blood pressure dip of the patient. However, in alternative embodiments, the blood pressure output signal may comprise information regarding a measure of a diurnal BP dip of the patient (e.g. obtained from a separate or external method).

The obtained measure of diurnal blood pressure dip of the patient is passed to step 220 which comprises determining a target exercise intensity for the patient based on the obtained measure of diurnal blood pressure dip. In this example, determining a target exercise intensity for the patient comprises: obtaining a waking blood pressure of the patient (e.g. from the obtained blood pressure of a signal); and calculating a target sleeping blood pressure for the patient based on the waking blood pressure and the measure of diurnal blood pressure dip of the patient (as obtained in step 210). The target exercise intensity is then calculated based on the target sleeping blood pressure. It will be appreciated that this process may be thought of as obtaining a BP differential which is then used to determine an optimal or preferred (i.e. target) sleeping BP for a patient. Using known or established relationships between exercise intensity and resultant BP variation, an exercise intensity for causing a BP variation to meet the optimal or preferred (i.e. target) sleeping BP can then be determined.

For instance, let one assume an example wherein the patient is a non-dipper, with a blood pressure dip of around 0% (i.e. blood pressure during the night is equal to daytime), and the daytime systolic BP is 130 mmHg. If it is then desired for the nocturnal BP to be about 15% lower, the preferred (i.e. target) sleeping BP may be calculated as 130-(0.15*130)=129 mmHg. This would indicate that, to have the desired nocturnal BP, the nocturnal BP should be 11 mmHg lower than the daytime value. If one then assumes that the systolic blood pressure drops with 1.5 mmHg per 10% of VO2max (as has been reported in some published studies), a target may be calculated as 10%*(11/1.5)=73% of VO2max. Calculation of the target exercise intensity can then be based on heart rate. For example, knowing the maximum heart rate, a target exercise intensity can be based on targeting 73% of the maximum heart rate. For the example, if one assumes that the user's maximum heart rate is 160 BPM, a target heart rat can be calculated as 160*73%=117 BPM. Thus, the target exercise intensity can be recommended as aerobic exercise (of about half an hour) at the target heart rate of 117 BPM. Of course, it will be appreciated that there may be many other ways to calculate target exercise intensity.

Step 230 comprises obtaining a measure of the circadian rhythm of the patient. Here, a sensor output signal is received from a sensor arrangement coupled to the patient. The sensor arrangement provides the sensor output signal which includes information relating to a value of at least one of: body temperature of the patient; activity of the patient; respiration rate of the patient; respiration rate variability of the patient; pulse rate of the patient; and pulse rate variability of the patient. The received sensor output signal is processed in accordance with one or more data processing algorithms to determine the measure of the circadian rhythm of the patient. Nonetheless, since various physiological values or parameters of the patient may thus be obtained for the purpose of determining the circadian rhythm of the patient, one or more different approaches to obtaining a measure of the circadian rhythm may be implemented, and such approaches may be combined for improved accuracy.

The obtained measure of the circadian rhythm of the patient is passed to step 235 which comprises determining target exercise timing for the patient based on the obtained measure of the circadian rhythm. In this example, determining a target exercise timing comprises comparing the measure of circadian rhythm with a threshold value (in step 240). Based on the result of the comparison, the target exercise timing may be determined to either be in a time window after waking or a time window before sleeping. More specifically, if, in step 240, it is determined that the patient is a morning person (i.e. the circadian rhythm peaks is earlier than a predetermined average represented by the threshold), the method proceeds to step 260 wherein the target exercise timing is determined to be early in the morning, shortly after waking. If, on the other hand, it is determined (in step 240) that the patient is an evening person (i.e. the circadian rhythm peaks later than the predetermined average represented by the threshold), the method proceeds to step 270 wherein the target exercise timing is determined to be in the evening prior to a predicted melatonin onset. In this regard, one can predict melatonin onset from the circadian rhythm, since it typically occurs 2 hours before sleep onset (which can be calculated based on the circadian rhythm).

Finally, in step 280, an output signal representative of the target exercise intensity and target exercise timing is generated. By way of example, the output signal may be a control signal for a graphical user interface and thus comprise instructions for controlling the graphical user interface to display information about the target exercise intensity and target exercise timing.

The method 200 may thus provide a signal comprising information for managing or controlling BP dipping through the combined timing and intensity of physical exercise.

From the above description of the flow diagram in Figure 2, it will be understood that embodiments may provide a concept managing or controlling BP functions of a patient.

Although the embodiment of Figure 2 has been described as comprising the steps of receiving output signals from separate sensing arrangements or methods, it should be understood that embodiments may comprise the additional step(s) of sensing a measure of BP dipping and/or circadian rhythm for a patient. By way of illustration, the flow diagram of Figure 2 has been illustrated as also comprising the optional steps of 290 and 295 as indicated by dotted lines. For instance, the method 200 may include the step 290 of sensing a measure of BP dipping for the patient (e.g. using a BP sensing arrangement). Also, the method 200 may include the step 295 of sensing a measure of the circadian rhythm of the patient (e.g. using an arrangement of sensors for detecting values such as body temperature, pulse, blood pressure, etc.).

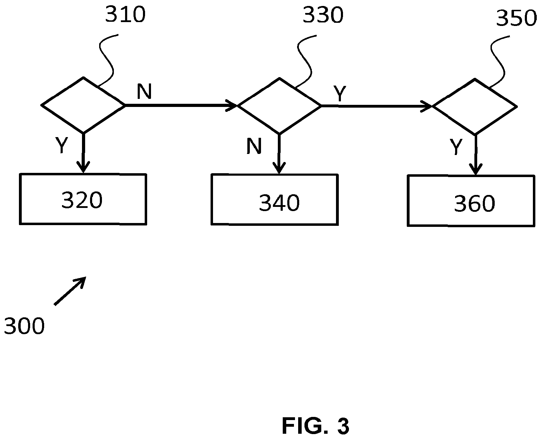

Turning now to Figure 3, there is depicted a flow diagram of an algorithm 300 according to an embodiment. The algorithm is adapted to prescribe the timing and intensity of exercise for restoring a deficiency in a blood pressure dip of a patient. By way of summary, the algorithm inputs comprise: (i) measured/detected internal circadian rhythm of the previous day(s); (ii) day-night rhythm of patient's time zone; and (iii) measured/detected BP dip of the previous day(s), and the algorithm output comprises a recommendation for the intensity and time of an exercise to be performed by the patient.

First, in step 310, it is determined if the measured/detected BP dip of the previous day(s) is healthy. By way of example, this comprises determining if the BP dip is between 10% and 20%. If it is determined that the BP dip is within the predetermined healthy range (e.g. between 10%-20%), the algorithm proceeds to step 320 wherein no specific recommendation is made (because the BP dip is considered healthy and no intervention in the patient's normal or current activities is deemed necessary).

If, however, it is determined (in step 310) that the BP dip is not within the predetermined healthy range (e.g. between 10%-20%), the algorithm proceeds to step 330 wherein it is determined if the measured internal circadian rhythm is within a predetermined acceptable range. By way of example, this comprises determining if the internal circadian rhythm is offset from the day-night rhythm of patient's time zone by less than 1 hour.

If it is determined (in step 330) that the internal circadian rhythm is offset from the day-night rhythm of patient's time zone by more than 1 hour (i.e. not within the predetermined acceptable range), the algorithm proceeds to step 340 wherein the timing of the exercise is recommended to be at dawn or dusk (at moderate intensity) so as to exploit the circadian mechanisms for the restoration of the BP dip.

If, however, it is determined (in step 330) that the internal circadian rhythm is offset from the day-night rhythm of patient's time zone by less than 1 hour (i.e. within the predetermined acceptable range), the algorithm proceeds to step 350 wherein it is determined if the BP dip is small. By way of example, this comprises determining (in step 350) if the BP dip is less than 10%.

If it is determined that the BP dip is small (e.g. less than 10%), the algorithm proceeds to step 360 wherein a target exercise intensity is calculated. In this example, the step 360 of calculating a target exercise intensity comprises the following steps and calculations:
(i) A value for the patient's their intended bedtime (IBT) is obtained, for example by prompting the patient to provide such information via a user interface;
(ii) A value of the user's current blood pressure (CBP) is obtained, for example by automatically operating a BP sensor worn by the user;
(iii) The timing of exercise is set to 3.5 hours before the IBT (in order to not interfere with sleep onset problems);
(iv) The deficiency in the BP dip is calculated as the post-exercise hypotension target (PEHT) in mmHg, e.g.: DEFIC = (15%-BPDIP)*CBP ; and
(v) The intensity of exercise to induce a post-exercise hypotension equal to PEHT is calculated. For example, for PEHT < 3 mmHg, exercise intensity=40%VO2M. For PEHT>3 mmHg, exercise intensity = argmin(70, 40+10*((PEHT-3)/1.5)).

From the above, it will be appreciated that proposed concepts may comprise the following components: BP dip monitoring (as an input); and internal circadian rhythm monitoring (as an input).

For BP dip monitoring, conventional or existing approaches may be implemented to monitor a patient's BP variations (e.g. nocturnal BP dip). By way of example, this can be an ambulatory blood pressure cuff that inflates automatically on a regular interval throughout the night or a smaller, less unobtrusive sensor integrated in a wearable computing device (such as a smartwatch or activity tracker for example)

For internal biological clock monitoring, various approaches may be implemented. An exemplary approach may, for example, employ core body temperature (CBT) monitoring, since CBT is regarded as an accurate reflection of the activity of the pacemaker a person's body clock. Measurements may be obtained using a temperature sensor (with IR-light, or another temperature measurement device) positioned in the ear, integrated in a wearable computing device, and/or using surrogate temperature measurements. Alternatively, or additionally, CBT may be obtained from a multitude of skin temperature sensors positioned on different parts of the body, preferably at distal locations. Alternatively, or additionally, measurements of physical activity can be used to determine circadian phase.

An exemplary approach for internal circadian rhythm monitoring may, for example, employ monitoring of pulse (e.g. inter-beat) intervals. Heart rate variability is a common measure of cardiovascular regulation by the autonomic nervous system. Using heart rate inter-beat intervals together with data from physical activity sensors, it can be possible to predict human circadian phase based on, as few as, only 24 hours of data. Such data can easily be recorded in ambulatory conditions. The heart rate intervals can be obtained in several ways, e.g. via ECG measurements using electrodes on the body or with photoplethysmography (PPG) sensors contained e.g. in spectacles, a wrist-worn device, or an in-ear device.

Yet another approach for internal circadian rhythm monitoring may comprise determining a current circadian phase using mathematical models of the human circadian pacemaker. Such models take ambient light levels and sleep-wake timing data as inputs and produce an estimate of the phase and amplitude of the circadian system. In propose embodiments, these models may additionally take the timing of the exercise into account.

It may be preferable for embodiments to recognize the phase of the current solar cycle. This can be easily done using an ambient light sensor built into any of the mentioned embodiments. Such a sensor will measure the variation of the ambient light intensity and is therefore able to determine episodes of dawn and dusk. In another embodiment, the phase of the current solar cycle can be determined based on the current geo-position of the user and the time-of-year. Both can be easily obtained using e.g. data from satellite positioning systems such as GPS or GLONASS, or data from other positioning systems using cellular techniques.

Proposed embodiments may be applicable for any personal health program targeting BP variations such as BP dipping. Disrupted BP variations (e.g. non-dipping) are a problem for shift work and/or sleep apnoea populations.

Is to be understood that proposed concepts may employ (or be employed on) at least one processor. Thus, there may be provided a computer program product downloadable from a communications network and/or stored on a computer readable medium and/or microprocessor-executable medium wherein the computer program product comprises computer program code instructions, which when executed by at least one processor, implement a method according to a proposed embodiment.

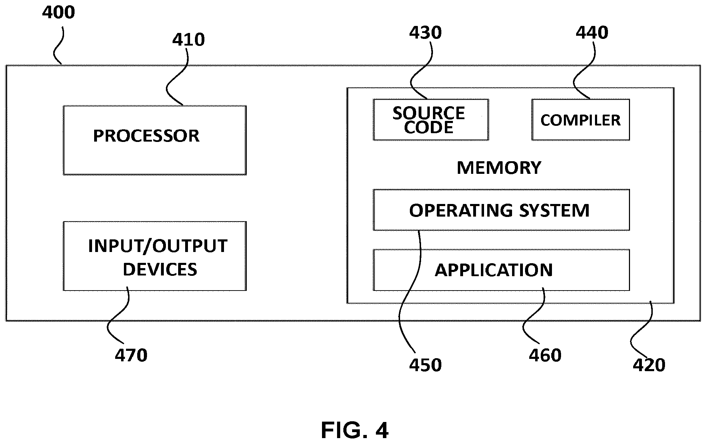

Figure 4 illustrates an example of a computer 400 within which one or more parts of an embodiment may be employed. Various operations discussed above may utilize the capabilities of the computer 400. For example, one or more parts of an apparatus for controlling blood pressure variation of a patient may be incorporated in any element, module, application, and/or component discussed herein.

The computer 400 includes, but is not limited to, PCs, workstations, laptops, PDAs, palm devices, servers, storages, and the like. Generally, in terms of hardware architecture, the computer 400 may include one or more processors 410, memory 420, and one or more I/O devices 470 (such as environmental sensors, infection source sensors, user susceptibility sensors, etc.) that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 410 is a hardware device for executing software that can be stored in the memory 420. The processor 410 can be virtually any custom made or commercially available processor, a central processing unit (CPU), a digital signal processor (DSP), or an auxiliary processor among several processors associated with the computer 400, and the processor 410 may be a semiconductor based microprocessor (in the form of a microchip) or a microprocessor.

The memory 420 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 420 may incorporate electronic, magnetic, optical, and/or other types of storage media. Note that the memory 420 can have a distributed architecture, where various components are situated remote from one another, but can be accessed by the processor 410.

The software in the memory 420 may include one or more separate programs, each of which comprises an ordered listing of executable instructions for implementing logical functions. The software in the memory 420 includes a suitable operating system (O/S) 450, compiler 440, source code 430, and one or more applications 460 in accordance with exemplary embodiments. As illustrated, the application 460 comprises numerous functional components for implementing the features and operations of the exemplary embodiments. The application 460 of the computer 400 may represent various applications, computational units, logic, functional units, processes, operations, virtual entities, and/or modules in accordance with exemplary embodiments, but the application 460 is not meant to be a limitation.

The operating system 450 controls the execution of other computer programs, and provides scheduling, input-output control, file and data management, memory management, and communication control and related services. It is contemplated by the inventors that the application 460 for implementing exemplary embodiments may be applicable on all commercially available operating systems.

Application 460 may be a source program, executable program (object code), script, or any other entity comprising a set of instructions to be performed. When a source program, then the program is usually translated via a compiler (such as the compiler 440), assembler, interpreter, or the like, which may or may not be included within the memory 420, so as to operate properly in connection with the O/S 450. Furthermore, the application 460 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, FORTRAN, COBOL, Perl, Java, ADA, .NET, and the like.

The I/O devices 470 may include input devices such as, for example but not limited to, a mouse, keyboard, scanner, microphone, camera, etc. Furthermore, the I/O devices 470 may also include output devices, for example but not limited to a printer, display, etc. Finally, the I/O devices 470 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 470 also include components for communicating over various networks, such as the Internet or intranet.

If the computer 400 is a PC, workstation, intelligent device or the like, the software in the memory 420 may further include a basic input output system (BIOS) (omitted for simplicity). The BIOS is a set of essential software routines that initialize and test hardware at startup, start the O/S 450, and support the transfer of data among the hardware devices. The BIOS is stored in some type of read-only-memory, such as ROM, PROM, EPROM, EEPROM or the like, so that the BIOS can be executed when the computer 400 is activated.

When the computer 400 is in operation, the processor 410 is configured to execute software stored within the memory 420, to communicate data to and from the memory 420, and to generally control operations of the computer 400 pursuant to the software. The application 460 and the O/S 450 are read, in whole or in part, by the processor 410, perhaps buffered within the processor 410, and then executed.

When the application 460 is implemented in software it should be noted that the application 460 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 460 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example, but not limited to, an electronic, magnetic, optical, electromagnetic, infrared, or semiconductor system, apparatus, device, or propagation medium.

Thus, there is proposed a concept for providing user guidance regarding both the timing and intensity of exercise at a personal level which is based on a measure of a sleeping BP variation and circadian rhythm of a person. By employing information regarding the circadian rhythm of the person timing of exercise may be optimized so as to ensure appropriate timing of its temporary anti-hypertensive benefits which can manage/control a sleeping BP variation (e.g. BP dipping).

At this point, it is noted that the above described embodiments are merely example embodiments and that several extensions thereto and/or variations may be made.

For example, the employment of several types of monitoring/sensing devices can be envisaged, including clip-on devices, smart textiles, smartwatches, mouth inserts, etc.

Data from the system may be combined with personal data on health and well-being to generate a personal profile exercise and sleep prescriptions or programs. Data may also be transmitted for the benefit of other peer users or patients interested in cardio-respiratory functions, and such data could serve as an input to health monitoring software.

Other suitable extensions and variations to the above disclosed embodiments will be apparent to the skilled person.

For example, embodiments may be adapted to implement flexible thresholds that can be adapted according to user and/or with respect to time. In this way, it may be possible to have more or less strict versions of algorithms used to create exercise plans or prescriptions.

A preferred implementation may be to only inform a user when a BP variation (e.g. BP dip) issue or anomaly is detected. This may help to ensure a seamless solution without inhibiting social interaction.

The proposed concept has the advantage that a network of portable computing device with monitoring and/or communication functions can be easily transformed into a BP variation management system.

Aspects of the present invention may be embodied as a BP variation management method or system at least partially embodied by a portable computing device or distributed over separate entities including a portable computing device. Aspects of the present invention may take the form of a computer program product embodied in one or more computer-readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. Such a system, apparatus or device may be accessible over any suitable network connection; for instance, the system, apparatus or device may be accessible over a network for retrieval of the computer readable program code over the network. Such a network may for instance be the Internet, a mobile communications network or the like. More specific examples (a non-exhaustive list) of the computer readable storage medium may include the following: an electrical connection having one or more wires, a portable computer diskette, a hard disk, a random access memory (RAM), a read-only memory (ROM), an erasable programmable read-only memory (EPROM or Flash memory), an optical fibre, a portable compact disc read-only memory (CD-ROM), an optical storage device, a magnetic storage device, or any suitable combination of the foregoing. In the context of the present application, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fibre cable, RF, etc., or any suitable combination of the foregoing.

Computer program code for carrying out the methods of the present invention by execution on the processor 111 may be written in any combination of one or more programming languages, including an object oriented programming language such as Java, Smalltalk, C++ or the like and conventional procedural programming languages, such as the "C" programming language or similar programming languages. The program code may execute entirely on the processor 111 as a stand-alone software package, e.g. an app, or may be executed partly on the processor 111 and partly on a remote server. In the latter scenario, the remote server may be connected to the portable computing device 100 through any type of network, including a local area network (LAN) or a wide area network (WAN), or the connection may be made to an external computer, e.g. through the Internet using an Internet Service Provider.

Aspects of the present invention are described above with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions to be executed in whole or in part on the data processor 111 of the system including portable computing device, such that the instructions create means for implementing the functions/acts specified in the flowchart and/or block diagram block or blocks. These computer program instructions may also be stored in a computer-readable medium that can direct the system including the portable computing device to function in a particular manner.

The computer program instructions may, for example, be loaded onto the portable computing device 100 to cause a series of operational steps to be performed on the portable computing device 100 and/or the server 120, to produce a computer-implemented process such that the instructions which execute on the portable computing device 100 and/or the server 120 provide processes for implementing the functions/acts specified in the flowchart and/or block diagram block or blocks. The computer program product may form part of a patient management system including a portable computing device.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A method for managing a blood-pressure variation of a patient, the method comprising, by a processing unit:
obtaining (210) a measure of a nocturnal blood pressure dip of the patient;
determining (220) a target exercise intensity for the patient based on the obtained measure of the nocturnal blood pressure dip including
- obtaining a waking blood pressure of the patient,
- calculating a target sleeping blood pressure for the patient based on the waking blood pressure and the measure of nocturnal blood pressure dip of the patient, and
- calculating the target exercise intensity based on the target sleeping blood pressure;
obtaining (230) a measure of the circadian rhythm of the patient;
determining (235) a target exercise timing for the patient based on the obtained measure of the circadian rhythm including
- determining a predicted timing of melatonin onset for the patient based on the measure of the circadian rhythm and processing the predicted timing of melatonin onset in accordance with one or more algorithms to calculate the target exercise timing for the patient; or
- comparing the measure of circadian rhythm with a threshold value and, based on the result of the comparison and determining the target exercise timing to either be in a time window after waking or a time window before sleeping; and
generating (280) an output signal representative of the target exercise intensity and target exercise timing.

2. The method of claim **1,** further comprising:
obtaining an updated measure of a nocturnal blood pressure dip of the patient, the updated measure being associated with a previously determined target exercise intensity; and
modifying the algorithm based on the updated measure of a nocturnal blood pressure dip of the patient and the previously determined target exercise intensity.

3. The method of any preceding claim, further comprising:
sensing, with a sensor arrangement worn by or coupled to the patient, a value of at least one of:
body temperature of the patient;
activity of the patient;
respiration rate of the patient;
respiration rate variability of the patient;
pulse rate of the patient; and
pulse rate variability of the patient; and
generating a sensor output signal based on the sensed value(s).

4. The method of claim 3, further comprising:
receiving, at a processing unit, a sensor output signal from the sensor arrangement; and
processing the received sensor output signal in accordance with one or more data processing algorithms to determine the measure of the circadian rhythm of the patient.

5. The method of any preceding claim, further comprising:
sensing (290), with a blood pressure sensing arrangement: a waking blood pressure of the patient when awake; and a sleeping blood pressure of the patient when asleep; and
generating a blood pressure output signal based on the sensed value(s).

6. The method of claim 5, further comprising:
receiving, at a processing unit, a blood pressure output signal from the blood pressure sensing arrangement; and
processing the received blood pressure output signal in accordance with one or more data processing algorithms to determine the measure of the nocturnal blood pressure dip of the patient.

7. A computer program product downloadable from a communications network and/or stored on a computer readable medium and/or microprocessor-executable medium wherein the computer program product comprises computer program code instructions, which when executed by at least one processor, implement a method as claimed in any preceding claim.

8. Apparatus (100) for managing a blood-pressure variation of a patient, the apparatus comprising:
an exercise intensity calculation unit (111) adapted to determine a target exercise intensity for the patient based on an obtained measure of nocturnal blood pressure dip of the patient including
- obtaining a waking blood pressure of the patient,
- calculating a target sleeping blood pressure for the patient based on the waking blood pressure and the measure of nocturnal blood pressure dip of the patient, and
- calculating the target exercise intensity based on the target sleeping blood pressure;
an exercise timing calculation unit adapted to determine a target exercise timing for the patient based on an obtained measure of the circadian rhythm of the patient including
- determining a predicted timing of melatonin onset for the patient based on the measure of the circadian rhythm and processing the predicted timing of melatonin onset in accordance with one or more algorithms to calculate the target exercise timing for the patient; or
- comparing the measure of circadian rhythm with a threshold value and, based on the result of the comparison and determining the target exercise timing to either be in a time window after waking or a time window before sleeping; and
an output unit (112, 114) adapted to generate an output signal representative of the target exercise intensity and target exercise timing.

9. The apparatus of claim 8, further comprising:
a sensor arrangement (10,110) adapted to be worn by or coupled to the patient, to sense a value of at least one of:
body temperature of the patient;
activity of the patient;
respiration rate of the patient;
respiration rate variability of the patient;
pulse rate of the patient;
a blood pressure of the patient; and
pulse rate variability of the patient; and
to generate a sensor output signal based on the sensed value(s).

10. A portable computing device comprising apparatus for managing a blood-pressure variation of a patient according to claim 8 or 9.

## Patentansprüche

1. Verfahren zum Managen einer Blutdruckschwankung eines Patienten, wobei das Verfahren umfasst, durch eine Verarbeitungseinheit:
Erhalten (210) einer Messung eines nächtlichen Blutdruckabfalls des Patienten;
Bestimmen (220) einer Ziel-Trainingsintensität für den Patienten auf Basis der erhaltenen Messung des nächtlichen Blutdruckabfalls, was beinhaltet
- Erhalten eines Wach-Blutdrucks des Patienten,
- Berechnen eines Ziel-Schlafblutdrucks für den Patienten auf Basis des Wach-Blutdrucks und der Messung des nächtlichen Blutdruckabfalls des Patienten, und
- Berechnen der Ziel-Trainingsintensität auf Basis des Ziel-Schlafblutdrucks;
Erhalten (230) einer Messung des zirkadianen Rhythmus des Patienten;
Bestimmen (235) eines Ziel-Trainingszeitpunkts für den Patienten auf Basis der erhaltenen Messung des zirkadianen Rhythmus, was beinhaltet
- Bestimmen eines vorhergesagten Zeitpunkts des Einsetzens von Melatonin für den Patienten auf Basis der Messung des zirkadianen Rhythmus, und Verarbeiten des vorhergesagten Zeitpunkts des Einsetzens von Melatonin gemäß einem oder mehreren Algorithmen, um den Ziel-Trainingszeitpunkt für den Patienten zu berechnen; oder
- Vergleichen der Messung des zirkadianen Rhythmus mit einem Schwellenwert und, auf Basis des Ergebnisses des Vergleichs, und Bestimmen des Ziel-Trainingszeitpunkts so, dass er sich entweder in einem Zeitfenster nach dem Aufwachen oder einem Zeitfenster vor dem Einschlafen befindet; und
Erzeugen (280) eines Ausgangssignals, das für die Ziel-Trainingsintensität und den Ziel-Trainingszeitpunkt repräsentativ ist.

2. Verfahren nach Anspruch 1, weiter umfassend:
Erhalten einer aktualisierten Messung eines nächtlichen Blutdruckabfalls des Patienten, wobei die aktualisierte Messung mit einer zuvor bestimmten Ziel-Trainingsintensität verknüpft ist; und
Modifizieren des Algorithmus auf Basis der aktualisierten Messung eines nächtlichen Blutdruckabfalls des Patienten und der zuvor bestimmten Ziel-Trainingsintensität.

3. Verfahren nach einem vorstehenden Anspruch, weiter umfassend:
Erfassen, mit einer Sensoranordnung, die vom Patienten getragen wird oder mit ihm gekoppelt ist, eines Werts von mindestens einem von:
Körpertemperatur des Patienten;
Aktivität des Patienten;
Atemfrequenz des Patienten;
Atemfrequenzvariabilität des Patienten;
Pulsfrequenz des Patienten; und
Pulsfrequenzvariabilität des Patienten; und
Erzeugen eines Sensorausgangssignals auf Basis des/der erfassten Werte(s).

4. Verfahren nach Anspruch 3, weiter umfassend:
Empfangen eines Sensorausgangssignals von der Sensoranordnung an einer Verarbeitungseinheit; und
Verarbeiten des empfangenen Sensorausgangssignals gemäß einem oder mehreren Datenverarbeitungsalgorithmen, um die Messung des zirkadianen Rhythmus des Patienten zu bestimmen.

5. Verfahren nach einem vorstehenden Anspruch, weiter umfassend:
Erfassen (290), mit einer Blutdrucksensoranordnung: eines Wach-Blutdrucks des Patienten, wenn er wach ist; und eines Schlafblutdrucks des Patienten, wenn er schläft; und
Erzeugen eines Blutdruckausgangssignals auf Basis des/der erfassten Werte(s).

6. Verfahren nach Anspruch 5, weiter umfassend:
Empfangen eines Blutdruckausgangssignals von der Blutdrucksensoranordnung an einer Verarbeitungseinheit; und
Verarbeiten des empfangenen Blutdruckausgangssignals gemäß einem oder mehreren Datenverarbeitungsalgorithmen, um die Messung des nächtlichen Blutdruckabfalls des Patienten zu bestimmen.

7. Computerprogrammprodukt, das aus einem Kommunikationsnetzwerk heruntergeladen werden kann und/oder auf einem computerlesbaren Medium und/oder Mikroprozessor-ausführbaren Medium gespeichert ist, wobei das Computerprogrammprodukt Computerprogrammcodeanweisungen umfasst, die, wenn sie von mindestens einem Prozessor ausgeführt werden, ein Verfahren nach einem vorstehenden Anspruch umsetzen.

8. Einrichtung (100) zum Managen einer Blutdruckschwankung eines Patienten, wobei die Einrichtung umfasst:
eine Trainingsintensitäts-Berechnungseinheit (111), die dazu ausgelegt ist, eine Ziel-Trainingsintensität für den Patienten auf Basis einer erhaltenen Messung eines nächtlichen Blutdruckabfalls des Patienten zu bestimmen, was beinhaltet
- Erhalten eines Wach-Blutdrucks des Patienten,
- Berechnen eines Ziel-Schlafblutdrucks für den Patienten auf Basis des Wach-Blutdrucks und der Messung des nächtlichen Blutdruckabfalls des Patienten, und
- Berechnen der Ziel-Trainingsintensität auf Basis des Ziel-Schlafblutdrucks;
eine Trainingszeitpunkt-Berechnungseinheit, die dazu ausgelegt ist, einen Ziel-Trainingszeitpunkt für den Patienten auf Basis einer erhaltenen Messung des zirkadianen Rhythmus des Patienten zu bestimmen, was beinhaltet
- Bestimmen eines vorhergesagten Zeitpunkts des Einsetzens von Melatonin für den Patienten auf Basis der Messung des zirkadianen Rhythmus, und Verarbeiten des vorhergesagten Zeitpunkts des Einsetzens von Melatonin gemäß einem oder mehreren Algorithmen, um den Ziel-Trainingszeitpunkt für den Patienten zu berechnen; oder
- Vergleichen der Messung des zirkadianen Rhythmus mit einem Schwellenwert und, auf Basis des Ergebnisses des Vergleichs, und Bestimmen des Ziel-Trainingszeitpunkts so, dass er sich entweder in einem Zeitfenster nach dem Aufwachen oder einem Zeitfenster vor dem Einschlafen befindet; und
eine Ausgabeeinheit (112, 114), die dazu ausgelegt ist, ein Ausgangssignal zu erzeugen, das für die Ziel-Trainingsintensität und den Ziel-Trainingszeitpunkt repräsentativ ist.

9. Einrichtung nach Anspruch 8, weiter umfassend:
eine Sensoranordnung (10,110), die dazu ausgelegt ist, vom Patienten getragen oder mit ihm gekoppelt zu werden, um einen Wert von mindestens einem zu erfassen von:
Körpertemperatur des Patienten;
Aktivität des Patienten;
Atemfrequenz des Patienten;
Atemfrequenzvariabilität des Patienten;
Pulsfrequenz des Patienten;
einem Blutdruck des Patienten; und
Pulsfrequenzvariabilität des Patienten; und
ein Sensorausgangssignal auf Basis des/der erfassten Werte(s) zu erzeugen.

10. Tragbare Rechenvorrichtung, die eine Einrichtung zum Managen einer Blutdruckschwankung eines Patienten nach Anspruch 8 oder 9 umfasst.

## Revendications

1. Procédé de gestion d'une variation de pression artérielle d'un patient, le procédé comprenant, au moyen d'une unité de traitement :
l'obtention (210) d'une mesure d'une baisse de pression artérielle nocturne du patient ;
la détermination (220) d'une intensité d'exercice cible pour le patient sur la base de la mesure obtenue de la baisse de pression artérielle nocturne, incluant
- l'obtention d'une tension artérielle au réveil du patient,
- le calcul d'une pression artérielle cible pendant le sommeil pour le patient sur la base de la pression artérielle au réveil et de la mesure de la baisse de pression artérielle nocturne du patient, et
- le calcul de l'intensité d'exercice cible sur la base de la pression artérielle cible pendant le sommeil ;
l'obtention (230) d'une mesure du rythme circadien du patient ;
la détermination (235) d'un moment d'exercice cible pour le patient sur la base de la mesure obtenue du rythme circadien incluant
- la détermination d'un moment prédit d'apparition de la mélatonine pour le patient sur la base de la mesure du rythme circadien et le traitement du moment prédit d'apparition de la mélatonine selon un ou plusieurs algorithmes pour calculer le moment d'exercice cible pour le patient ; ou
- la comparaison de la mesure du rythme circadien avec une valeur seuil et, sur la base du résultat de la comparaison, la détermination du moment d'exercice cible pour qu'il se situe soit dans une fenêtre temporelle après le réveil, soit dans une fenêtre temporelle avant le sommeil ; et
la génération (280) d'un signal de sortie représentatif de l'intensité d'exercice cible et du moment d'exercice cible.

2. Procédé selon la revendication 1, comprenant en outre :
l'obtention d'une mesure actualisée d'une baisse de pression artérielle nocturne du patient, la mesure actualisée étant associée à une intensité d'exercice cible préalablement déterminée ; et
la modification de l'algorithme sur le base de la mesure actualisée d'une baisse de pression artérielle nocturne du patient et de l'intensité d'exercice cible préalablement déterminée.

3. Procédé selon une quelconque revendication précédente, comprenant en outre :
la détection, à l'aide d'un agencement de capteur porté par le patient ou couplé à celui-ci, d'une valeur d'au moins l'une parmi :
la température corporelle du patient ;
l'activité du patient ;
la fréquence respiratoire du patient ;
la variabilité de la fréquence respiratoire du patient ;
la fréquence du pouls du patient ; et
la variabilité de la fréquence du pouls du patient ; et
la génération d'un signal de sortie de capteur sur la base de la ou des valeurs détectées.

4. Procédé selon la revendication 3, comprenant en outre :
la réception, au niveau d'une unité de traitement, d'un signal de sortie de capteur en provenance de l'agencement de capteur ; et
le traitement du signal de sortie de capteur reçu selon un ou plusieurs algorithmes de traitement de données pour déterminer la mesure du rythme circadien du patient.

5. Procédé selon une quelconque revendication précédente, comprenant en outre :
la détection (290), avec un agencement de détection de la pression artérielle : d'une pression artérielle au réveil du patient lorsqu'il est réveillé ; et d'une pression artérielle de sommeil du patient lorsqu'il est endormi ; et
la génération d'un signal de sortie de pression artérielle sur la base de la ou des valeurs détectées.

6. Procédé selon la revendication 5, comprenant en outre :
la réception, au niveau d'une unité de traitement, d'un signal de sortie de pression artérielle en provenance de l'agencement de détection de pression artérielle ; et
le traitement du signal de sortie de pression artérielle reçu selon un ou plusieurs algorithmes de traitement de données pour déterminer la mesure de la baisse de pression artérielle nocturne du patient.

7. Produit de programme informatique téléchargeable à partir d'un réseau de communication et/ou stocké sur un support lisible par ordinateur et/ou un support exécutable par microprocesseur, dans lequel le produit de programme informatique comprend des instructions de code de programme informatique qui, lorsqu'elles sont exécutées par au moins un processeur, mettent en œuvre un procédé selon une quelconque revendication précédente.

8. Appareil (100) de gestion d'une variation de pression artérielle d'un patient, l'appareil comprenant :
une unité de calcul d'intensité d'exercice (111) adaptée pour déterminer une intensité d'exercice cible pour le patient sur la base d'une mesure obtenue de la baisse de pression artérielle nocturne du patient incluant
- l'obtention d'une tension artérielle au réveil du patient,
- le calcul d'une pression artérielle cible pendant le sommeil pour le patient sur la base de la pression artérielle au réveil et de la mesure de la baisse de pression artérielle nocturne du patient, et
- le calcul de l'intensité d'exercice cible sur la base de la pression artérielle cible pendant le sommeil ;
une unité de calcul du moment d'exercice adaptée pour déterminer un moment d'exercice cible pour le patient sur la base d'une mesure obtenue du rythme circadien du patient incluant
- la détermination d'un moment prédit d'apparition de la mélatonine pour le patient sur la base de la mesure du rythme circadien et le traitement du moment prédit d'apparition de la mélatonine selon un ou plusieurs algorithmes pour calculer le moment d'exercice cible pour le patient ; ou
- la comparaison de la mesure du rythme circadien avec une valeur seuil et, sur la base du résultat de la comparaison, la détermination du moment d'exercice cible pour qu'il se situe soit dans une fenêtre temporelle après le réveil, soit dans une fenêtre temporelle avant le sommeil ; et
une unité de sortie (112, 114) conçue pour générer un signal de sortie représentatif de l'intensité d'exercice cible et du moment d'exercice cible.

9. Appareil selon la revendication 8, comprenant en outre :
un agencement de capteur (10, 110) adapté pour être porté par le patient ou couplé à celui-ci, pour détecter une valeur d'au moins l'une parmi :
la température corporelle du patient ;
l'activité du patient ;
la fréquence respiratoire du patient ;
la variabilité de la fréquence respiratoire du patient ;
la fréquence du pouls du patient ;
la pression artérielle du patient ; et
la variabilité de la fréquence du pouls du patient ; et
pour générer un signal de sortie de capteur sur la base de la ou des valeurs détectées.

10. Dispositif informatique portable comprenant un appareil de gestion d'une variation de pression artérielle d'un patient selon la revendication 8 ou 9.
